# EUROPEAN PATENT APPLICATION

(11) **EP 0 692 506 A2**
(43) Date of publication of application: **17.01.1996**
(21) Application number: 95110951.1
(22) Date of filing: 13.07.1995
(51) Int. Cl.: C08G 18/00

(54) **Thermo-sensitive polyether polyurethane, production method thereof and thermo-sensitive composition**

(30) Priority: 14.07.1994 JP 185340/94
(71) Applicant: MIYOSHI YUSHI KABUSHIKI KAISHA, Katsushika-ku Tokyo 124 (JP)
(72) Inventor: Tsukanome, Masayoshi, c/o Miyoshi Yushi K.K., Tokyo (JP); Kuroda, Iwao, Miyoshi Yushi Kabushiki Kaisha, Iwakura-shi, Aichi-ken (JP); Kamio, Katsuhisa, Miyoshi Yushi Kabushiki Kaisha, Iwakura-shi, Aichi-ken (JP); Kawashima, Masatake Miyoshi Yushi Kabushiki Kaisha, Iwakura-shi, Aichi-ken (JP); Moriya, Masafumi, Miyoshi Yushi K.K., Tokyo (JP)
(74) Representative: Müller-Gerbes, Margot, Dipl.-Ing.

(57) **Abstract**

Disclosed herein is a thermosensitive polyether polyurethane obtained from a polyoxyalkylene glycol and a diisocyanate compound. The polyether urethane contains polyoxyethylene chain(s), and polyoxypropylene chain(s) having an average molecular weight of 700-20,000 in a weight ratio of 95:5 to 50:50. A method of producing the thermosensitive polyether polyurethane is also disclosed, wherein polyoxyethylene-polyoxypropylene glycol containing polyoxyethylene chain(s), and polyoxypropylene chain(s) having an average molecular weight of 700-20,000 in a weight ratio of 95:5 to 50:50, or a mixture of polyoxyethylene glycol, and polyoxypropylene glycol having an average molecular weight of 700-20,000 mixed in such amounts that a weight ratio of polyoxyethylene chain(s) to polyoxypropylene chain(s) in the mixture is 95:5 to 50:50 is reacted with a diisocyanate compound, or wherein a mixture of polyoxyethylene-polyoxypropylene glycol containing polyoxyethylene chain(s), and polyoxypropylene chain(s) having an average molecular weight of 700-20,000, and polyoxyethylene glycol and/or polyoxypropylene glycol having an average molecular weight of 700-20,000 mixed in such amounts that a weight ratio of polyoxyethylene chain(s) to polyoxypropylene chain(s) in the mixture is 95:5 to 50:50 is reacted with a diisocyanate compound. The diisocyanate compound is reacted in such an amount that isocyanate groups amount to 0.5-1.5 molar equivalents to hydroxyl groups in the polyoxyalkylene glycol or the mixture.

## Description

### BACKGROUND OF THE INVENTION

### Field of the Invention:

The present invention relates to a thermosensitive polyether polyurethane, a production method thereof and a thermosensitive composition. More particularly, this invention relates to a polyether polyurethane, which has special properties that its aqueous solution reversibly undergoes a change of state in which the aqueous solution remains uniform and transparent at temperatures not higher than a certain temperature, while it gels when heated to a temperature higher than the certain temperature, and water is separated from the gel when heated further, and uses, as thermosensitive compositions, in applications such as thickeners, gelatinizers, adsorbents, resisting agents, resist printing pastes, printing pastes, release-controlled agents, cosmetic compositions, light-screening materials, adhesives or pressure-sensitive adhesives, dispersion stabilizers, fragrances, heat-regenerating media for cooling, discharge printing pastes and printing adhesives, and a production process thereof. The present invention further relates to a thermosensitive composition comprising an aqueous solution of the thermosensitive polyether polyurethane as a principal component.

### Description of the Background Art:

In recent years, there have been developed thermosensitive polymers having such properties that they are hydrophilic at temperatures not higher than a certain temperature, and their aqueous solutions remain uniform and transparent, while they turn hydrophobic when heated to a temperature higher than the certain temperature, and the aqueous solutions gel, or they are separated from the aqueous phase. Such polymers are expected to be applicable to uses such as thickeners, gelatinizers and adsorbents.

As such thermosensitive polymers, there have been known polymers of acrylamide derivatives, such as polymers of N-isopropylacrylamide and N-isopropylmethacrylamide (Japanese Patent Application Laid-Open Nos. 174408/1983 and 215413/1983), a polymer of N-isopropylacrylamide (Japanese Patent Application Laid-Open No. 201810/1983), polymers of N-methoxypropylacrylamide (Japanese Patent Application Laid-Open Nos. 225203/1986, 117016/1888 and 117017/1988), a polymer of N-ethoxyethyl(meth)acrylamide (Japanese Patent Application Laid-Open No. 91509/1987), a polymer of N-tetrahydrofurfurylacrylamide (Japanese Patent Application Laid-Open No. 148504/1987), a polymer of N-methyl-N-isopropylacrylamide (Japanese Patent Application Laid-Open No. 243608/1987), a polymer of N-methyl-N-n-propylacrylamide (Japanese Patent Application Laid-Open No. 243609/1987), a polymer of N-methoxyethoxypropyl(meth)acrylamide (Japanese Patent Application Laid-Open No. 70511/1989), a polymer of N-1-methyl-2-methoxyethyl (meth)acrylamide (Japanese Patent Application Laid-Open No. 295613/1988), a polymer of N-1-methoxymethylpropyl(meth)acrylamide (Japanese Patent Application Laid-Open No. 11110/1989), a polymer of N,N-di(2-methoxyethyl)acrylamide (Japanese Patent Application Laid-Open No. 249815/1989), a polymer of N-(2-methoxyethyl)-N-n-propylacrylamide (Japanese Patent Application Laid-Open No. 147609/1990), a polymer of N-(2-methoxyethyl)-N-isopropylacrylamide (Japanese Patent Application Laid-Open No. 214705/1990) and an ultra-high-molecular-weight polyacrylamide (Japanese Patent Application Laid-Open No. 114011/1992). There have also been known polymers of acrylamide derivatives, such as a copolymer of an acrylamide type vinyl polymer and a water-insoluble vinyl compound (Japanese Patent Application Laid-Open No. 75682/1990), a polymer of N-8-acryloyl-1,4-dioxa-8-azaspiro[4,5]decane (Japanese Patent Application Laid-Open No. 178111/1988), a polymer of a reaction product of (meth)acryloyl chloride, 3-methoxypropylamine and triethylamine (Japanese Patent Application Laid-Open No. 241007/1988), a polymer of a reaction product of (meth)acryloyl chloride, N-(2-methoxyethyl)ethylamine and triethylamine (Japanese Patent Application Laid-Open No. 129209/1990), a polymer of an oxyalkylene ether of a polysaccharide and a vinyl monomer (Japanese Patent Application Laid-Open No. 259084/1989), a water-soluble vinyl polymer containing, as a polymeric component, at least 50% of an ester of an alkylene oxide adduct of an active hydrogen compound having a nitrogen-containing ring with a vinylcarboxylic acid (Japanese Patent Application Laid-Open No. 9848/1994), polyvinyl methyl ether and methylcellulose.

However, these conventionally-known thermosensitive polymers have involved drawbacks that it is difficult to control a temperature at which their aqueous solutions change from a fluid state to a gelled state (hereinafter, this change of state is referred to as "phase transition", and the temperature at which the phase transition occurs is referred to as "phase transition temperature"), and/or that difficulties are encountered on their production. For example, the N-substituted acrylamide polymers and copolymers exhibit individual fixed phase transition temperatures according to the kinds of the polymers. Therefore, they are accompanied by a problem that the phase transition temperature may not be optionally preset in only one kind of polymer. In addition, all the conventionally-known thermosensitive polymers have been derived from vinyl monomers and hence involved a drawback that their polymerization degrees are difficult to control, and so they cannot be provided as polymers having a molecular weight within a fixed range. If the molecular weight of the polymer is too low, it cannot exhibit good hydrophobicity even when its aqueous solution is heated to its phase transition temperature or higher. On the other hand, if the molecular weight of the polymer is too high, the polymer cannot clearly exhibit the thickening effect at temperatures lower than its phase transition temperature. Besides, there is a problem that the change of state becomes unclear if the molecular weight distribution is wide.

### SUMMARY OF THE INVENTION

The present invention has been made in view of the foregoing circumstances, and has as its object the provision of a thermosensitive polyether polyurethane which has properties that it exhibits good water-solubility and is prepared into a uniform and transparent aqueous solution at temperatures lower than its phase transition temperature, and the solution reversibly turns gel or state that water is separated therefrom at the phase transition temperature or higher, and in which the phase transition temperature can be optionally preset, and a production method thereof.

Another object of the present invention is to provide a thermosensitive composition comprising an aqueous solution of this thermosensitive polyether polyurethane as a principal component.

The present inventors have carried out an extensive investigation with a view toward solving the above-described problems. As a result, it has been found that an aqueous solution of a polyether polyurethane obtained from a specific polyoxyalkylene glycol and a diisocyanate increases in viscosity as its temperature rises in a temperature range lower than its phase transition temperature, clearly changes from liquid to gel when the temperature exceeds the phase transition temperature, turns state that water is separated therefrom when further heated (this temperature is referred to as "phase separation temperature"), and moreover reversibly exhibits such change of state, and that the phase transition temperature and phase separation temperature can be optionally preset with ease, thus leading to completion of the present invention.

According to the present invention, there is thus provided a thermosensitive polyether polyurethane obtained from a polyoxyalkylene glycol and a diisocyanate compound, characterized in that the polyurethane contains polyoxyethylene chain(s), and polyoxypropylene chain(s) having an average molecular weight of 700-20,000 in a weight ratio of 95:5 to 50:50.

According to the present invention, there is also provided a method of producing a thermosensitive polyether polyurethane, characterized in that polyoxyethylenepolyoxypropylene glycol containing polyoxyethylene chain(s), and polyoxypropylene chain(s) having an average molecular weight of 700-20,000 in a weight ratio of 95:5 to 50:50 is reacted with a diisocyanate compound in such an amount that isocyanate groups amount to 0.5-1.5 molar equivalents to hydroxyl groups in the polyoxyethylenepolyoxypropylene glycol.

According to the present invention, there is further provided a method of producing a thermosensitive polyether polyurethane, characterized in that a mixture of polyoxyethylene glycol, and polyoxypropylene glycol having an average molecular weight of 700-20,000 mixed in such amounts that a weight ratio of polyoxyethylene chain(s) to polyoxypropylene chain(s) in the mixture is 95:5 to 50:50 is reacted with a diisocyanate compound in such an amount that isocyanate groups amount to 0.5-1.5 molar equivalents to hydroxyl groups in the mixture.

According to the present invention, there is still further provided a method of producing a thermosensitive polyether polyurethane, characterized in that a mixture of polyoxyethylene-polyoxypropylene glycol containing polyoxypropylene chain(s) having an average molecular weight of 700-20,000, and polyoxyethylene glycol and/or polyoxypropylene glycol having an average molecular weight of 700-20,000 mixed in such amounts that a weight ratio of polyoxyethylene chain(s) to polyoxypropylene chain(s) in the mixture is 95:5 to 50:50 is reacted with a diisocyanate compound in such an amount that isocyanate groups amount to 0.5-1.5 molar equivalents to hydroxyl groups in the mixture.

According to the present invention, there is yet still further provided a thermosensitive composition comprising an aqueous solution of the thermosensitive polyether polyurethane described above as a principal component.

The thermosensitive polyether polyurethane according to the present invention contains polyoxyethylene chain(s), and polyoxypropylene chain(s) having an average molecular weight of 700-20,000 in specific proportions. Therefore, its aqueous solution remains uniform and transparent and increases in viscosity as its temperature rises in a temperature range not higher than a certain temperature, clearly turns gel when heated beyond the certain temperature, and exhibits a state that water is separated therefrom when further heated. This change of state can be reversibly caused. Besides, when a method in which a polyoxyalkylene glycol prepared in such a manner that the ratio of polyoxyethylene chain(s) to polyoxypropylene chain(s) having an average molecular weight of 700-20,000 is within a specific range is use, and the polyoxyalkylene glycol is reacted with a diisocyanate in a specific proportion is adopted, a water-soluble polyether polyurethane having the particular function described above can be provided with ease. The thermosensitive composition comprising, as a principal component, the thermosensitive polyether polyurethane according to the present invention has such advantageous effects as it can be provided as a composition having a phase transition temperature and a phase separation temperature necessary for the end application intended by adjusting the molecular weight of the polyoxypropylene chain and the ratio of the polyoxyethylene chain(s) to the polyoxypropylene chain(s).

The above and other objects, features and advantages of the present invention will become apparent from the following description of the invention and the appended claims.

### DETAILED DESCRIPTION OF THE PREFERRED EMBODIMENTS

The polyether polyurethane according to the present invention is obtained by a method in which polyoxyethylene-polyoxypropylene glycol containing polyoxyethylene chain(s), and polyoxypropylene chain(s) having an average molecular weight of 700-20,000 in a weight ratio of 95:5 to 50:50, or a mixture of polyoxyethylene glycol and polyoxypropylene glycol having an average molecular weight of 700-20,000 mixed in such amounts that a weight ratio of polyoxyethylene chain(s) to polyoxypropylene chain(s) in the mixture is 95:5 to 50:50 is reacted with a diisocyanate compound, or a method in which a mixture of polyoxyethylene-polyoxypropylene glycol containing polyoxyethylene chain(s), and polyoxypropylene chain(s) having an average molecular weight of 700-20,000, and polyoxyethylene glycol and/or polyoxypropylene glycol having an average molecular weight of 700-20,000 mixed in such amounts that a weight ratio of polyoxyethylene chain(s) to polyoxypropylene chain(s) in the mixture is 95:5 to 50:50 is reacted with a diisocyanate compound. In this reaction, the polyoxyalkylene glycol is reacted with the diisocyanate compound in such an amount that isocyanate groups amount to 0.5-1.5 molar equivalents to hydroxyl groups in the polyoxyalkylene glycol. The thermosetting composition according to the present invention comprises, as a principal component, an aqueous solution of the thermosensitive polyether polyurethane.

The polyether polyurethane according to the present invention is hydrated at its ether linkage sites and dissolved in water when its aqueous solution is prepared. When this aqueous solution is heated, the polyether polyurethane first undergoes dehydration at its polyoxypropylene chain segment and turns hydrophobic, and so adjacent polyoxypropylene chains associate with each other by hydrophobic bonding, resulting in gelation. When further heated, the polyurethane also undergoes dehydration at polyoxyethylene chains bonded to the polyoxypropylene chains, and so the whole molecule turns hydrophobic to separate from water. That is, the same phenomenon as the clouding point phenomenon characteristic of nonionic surfactants occurs, and the aqueous solution becomes cloudy. This allows the polyether polyurethane according to the present invention to optionally preset the phase transition temperature by appropriately changing the weight ratio between the polyoxyethylene chain(s) and the polyoxypropylene chain(s), which make up the molecule of the polyether polyurethane according to the present invention, and the average molecular weight of the polyoxypropylene chain(s). For example, in the case of a polyether polyurethane containing polyoxypropylene chain(s) having an average molecular weight of 700, the phase transition temperature is about 30°C if the weight ratio of the polyoxyethylene chain(s) to the polyoxypropylene chain(s) is 50, while the phase transition temperature is about 90°C if the weight ratio is 95. As described above, the phase transition temperature proportionally rises as the proportion of the polyoxyethylene chain(s) in the polyether polyurethane increases. Therefore, the phase transition temperature can be preset to the desired temperature by changing the ratio of the polyoxyethylene chain(s) to the polyoxypropylene chain(s). Besides, the use of a polyoxyalkylene glycol containing polyoxypropylene chain(s) higher in average molecular weight can lower the phase transition temperature even in the same weight ratio as described above because the hydrophobicity or association strength becomes high.

The polyether polyurethane according to the present invention is obtained from a polyoxyalkylene glycol and a diisocyanate compound, and must contain polyoxyethylene chain(s) and polyoxypropylene chain(s) in its molecule. The average molecular weight of the polyoxypropylene chain(s) making up the polyether polyurethane is 700-20,000. When used as a thermosensitive composition, the inclusion of the polyoxypropylene chain(s) having such an average molecular weight allows an aqueous solution of the polyether polyurethane to exhibit the effect of increasing its viscosity as the temperature thereof rises, become a clear gel state when heated beyond its phase transition temperature and separate water therefrom when further heated. Even if the polyurethane is a polyether polyurethane obtained from a polyoxyalkylene glycol and a diisocyanate compound, its aqueous solution neither shows a tendency to increase its viscosity nor exhibits a clear gel state if the average molecular weight of the polyoxypropylene chain(s) making up its molecule is lower than 700. On the other hand, if the average molecular weight of the polyoxypropylene chain(s) exceeds 20,000, the resulting polyether polyurethane becomes poor in water-solubility. Therefore, it is not preferable to use any polyoxyalkylene glycol containing polyoxypropylene chain(s) having any average molecular weight outside the above-described range. In the polyether polyurethane according to the present invention, the average molecular weight of the polyoxypropylene chain(s) is preferably 2,000-5,000.

The polyether polyurethane according to the present invention contains polyoxyethylene chain(s) and polyoxypropylene chain(s) in a weight ratio of 95:5 to 50:50, thereby exhibiting good properties as to the thickening, phase transition and phase separation. With respect to the weight ratio of the polyoxyethylene chain(s) to the polyoxypropylene chain(s) in the polyether polyurethane molecule, if the proportion of the polyoxyethylene chain(s) exceeds 95, namely, the proportion of the polyoxypropylene chain(s) is lower than 5, its aqueous solution neither shows a tendency to increase its viscosity nor exhibits a clear gel state. On the other hand, if the proportion of the polyoxyethylene chain(s) is lower than 50, namely, the proportion of the polyoxypropylene chain(s) exceeds 50, the resulting polyurethane becomes poor in water-solubility. Therefore, it is not preferable to use any polyoxyalkylene glycol containing polyoxyethylene chain(s) and polyoxypropylene chain(s) in any weight ratio outside the above-described range. The weight ratio of the polyoxyethylene chain(s) to the polyoxypropylene chain(s) in the polyether polyurethane according to the present invention is preferably 90:10 to 60:40.

In order to obtain the polyether polyurethane according to the present invention, polyoxyethylenepolyoxypropylene glycol containing polyoxyethylene chain(s), and polyoxypropylene chain(s) having an average molecular weight of 700-20,000 in a weight ratio of 95:5 to 50:50, or a mixture of polyoxyethylene glycol, and polyoxypropylene glycol having an average molecular weight of 700-20,000 mixed in such amounts that a weight ratio of polyoxyethylene chain(s) to polyoxypropylene chain(s) in the mixture is 95:5 to 50:50 is reacted with a diisocyanate compound. Alternatively, the polyether polyurethane may also be obtained by mixing polyoxyethylene-polyoxypropylene glycol containing polyoxypropylene chain(s) having an average molecular weight of 700-20,000 and polyoxyethylene glycol and/or polyoxypropylene glycol having an average molecular weight of 700-20,000 in such amounts that a weight ratio of polyoxyethylene chain(s) to polyoxypropylene chain(s) in the mixture is 95:5 to 50:50, and reacting the resultant mixture with a diisocyanate compound.

As the polyoxyethylene-polyoxypropylene glycol, there may be used any of an adduct of polyoxyethylene glycol with propylene oxide and an adduct of polyoxypropylene glycol with ethylene oxide. Those obtained by further adding propylene oxide and/or ethylene oxide to these adducts may be used.

Examples of the diisocyanate compound include aliphatic diisocyanates such as ethylene diisocyanate, hexamethylene diisocyanate and decamethylene diisocyanate, alicyclic diisocyanates such as bis(isocyanomethyl)cyclohexane, hydrogenated diphenylmethane diisocyanate and hydrogenated tolylene diisocyanate, and aromatic diisocyanates such as xylylene diisocyanate, phenylene diisocyanate, tolylene diisocyanate, diphenylmethane diisocyanate, dimethyldiphenylene diisocyanate and naphthylene diisocyanate. These compounds may be used either singly or in any combination thereof. Of these, aliphatic diisocyanates and/or alicyclic diisocyanates are preferred because a polyether polyurethane excellent in light fastness can be provided.

The thermosensitive polyether polyurethane according to the present invention is preferably soluble in water. In order to obtain a polyether polyurethane excellent in water-solubility, a polyoxyalkylene glycol is reacted with a diisocyanate compound in such an amount that isocyanate groups amount to 0.5-1.5 molar equivalents to hydroxyl groups in the polyoxyalkylene glycol. The thermosensitive polyether polyurethane thus obtained exhibits good water-solubility and thickening effect in an aqueous solution. If the amount of the diisocyanate compound is less than 0.5 molar equivalent in the above reaction, the resulting polyurethane becomes insufficient in viscosity increase when prepared into an aqueous solution, and such an aqueous solution is difficult to gel. On the other hand, if the amount exceeds 1.5 molar equivalents, crosslinking occurs due to an excess amount of the diisocyanate, and so the resulting polyether polyurethane becomes low in water-solubility. Therefore, it is not preferable to use the diisocyanate compound in any amount outside the above range. A preferable proportion of the diisocyanate compound in the reaction with the polyoxyalkylene glycol is such that isocyanate groups amount to 0.8-1.2 molar equivalents to hydroxyl groups in the polyoxyalkylene glycol.

The urethanation reaction of the polyoxyalkylene glycol with the diisocyanate compound may be carried out by adding the diisocyanate compound to the polyoxyalkylene glycol at 30-250°C. In order to lessen the amount of unreacted diisocyanate compound and prevent the decomposition of the polyoxyalkylene glycol, it is preferable to conduct the reaction at 50-200°C. The urethanation reaction may be conducted either in the presence of a solvent or without using any solvent. When a solvent is used in the urethanation reaction as needed, a solvent inert to the isocyanate and hydroxyl groups is used as the solvent. Examples of the solvent used include aromatic solvents such as benzene, toluene and xylene, ketone solvents such as tetrahydrofuran, acetone and methyl ethyl ketone, and chlorinated solvents such as chlorobenzene, dichloroethane and carbon tetrachloride.

The urethanation reaction is preferably performed in the presence of a catalyst. As the catalyst, there may be used any known catalysts for preparation of urethane, for example, tertiary amines such as trimethylamine and triethylamine, organotin compounds such as tributyltin acetate, dibutyltin dilaurate and tin(II) octanoate, and organobismuth compounds. These catalysts may be used in an amount of about 5-1,000 ppm based on the total amount of the polyoxyalkylene glycol and the diisocyanate compound. In order to prevent the decomposition and deterioration of the polyoxyalkylene glycol, it is further preferable to conduct the urethanation reaction in the presence of an antioxidant.

An aqueous solution of the polyether polyurethane according to the present invention has special properties that it reversibly undergoes a change of state in which the solution remains uniform and transparent at temperatures not higher than a certain temperature, while it gels when heated to a temperature higher than the certain temperature, and water is separated from the gel when heated further, and can use, as thermosensitive compositions, in various applications such as thickeners, gelatinizers, adsorbents, resisting agents, resist printing pastes, printing pastes, release-controlled agents, cosmetic compositions, light-screening materials, adhesives or pressure-sensitive adhesives, dispersion stabilizers, fragrances, heat-regenerating media for cooling, discharge printing pastes and printing adhesives. The thermosensitive composition comprising, as a principal component, the aqueous solution of the polyether polyurethane according to the present invention may contain various additives, colorants and the like as necessary for the end application intended.

The present invention will hereinafter be described in more detail by the following examples. However, this invention is not limited to these examples, and variations may be made within limits not departing from the spirit and scope of the invention.

### Examples 1-3 and Comparative Examples 1-2:

Respective 300-ml four-necked flasks were charged with 100 g of their corresponding polyoxyalkylene glycols shown in Table 1, hexamethylene diisocyanate in an amount of 1 molar equivalent to hydroxyl groups in the polyoxyalkylene glycol, 50 mg of an antioxidant (IRGANOX 245) and 50 mg of dibutyltin dilaurate to conduct reaction 50°C for 30 hours, thereby obtaining respective polyether polyurethanes. Water was added to the resultant polyether polyurethanes to prepare 5% aqueous solutions. Each of the aqueous solutions was heated at a rate of 1°C/min from room temperature to measure a phase transition temperature at which the flowability of the aqueous solution was lost, and the aqueous solution turned gel. Incidentally, the polyether polyurethane obtained in Comparative Example 1 was insoluble in water of 0°C or higher, and so no aqueous solution was obtained.

The polyoxyalkylene glycols used in these examples and comparative examples were four kinds of polyoxyethylene-polyoxypropylene glycol [four adducts of polyoxypropylene glycol with 30 moles of ethylene oxide (hereinafter abbreviated as "EO"), with 55 moles of EO, with 130 moles of EO and with 520 moles of EO] obtained by adding EO to polyoxypropylene glycol having an average molecular weight of 2,000, and polyoxyethylene glycol having an average molecular weight of 8,000. The kinds of the polyoxyalkylene glycols used in Examples 1-3 and Comparative Examples 1-2, the contents of polyoxypropylene chain(s) in the polyoxyalkylene glycols, the phase transition temperatures of the resulting polyether polyurethanes and the weight ratios (PEC:PPC) of the polyoxyethylene chain(s) (hereinafter abbreviated as "PEC") to the polyoxypropylene chain(s) (hereinafter abbreviated as "PPC") in the polyether polyurethanes are shown in Table 1.

All the aqueous solutions of the polyether polyurethanes obtained in Examples 1-3 were transparent and fluid solutions having a viscosity of 5-30 cP at room temperature and exhibited a clear gel state when heated beyond their phase transition temperatures, respectively. When the gelled solutions were cooled to room temperature, all of them returned to the state of fluid solution before the heating. Besides, the aqueous solutions were recognized to show a tendency for the phase transition temperature to lower as the proportion of the polyoxypropylene chain(s) increased. To the contrary, the aqueous solution of the polyether polyurethane obtained in Comparative Example 2 did not gel even when heated to 95°C.

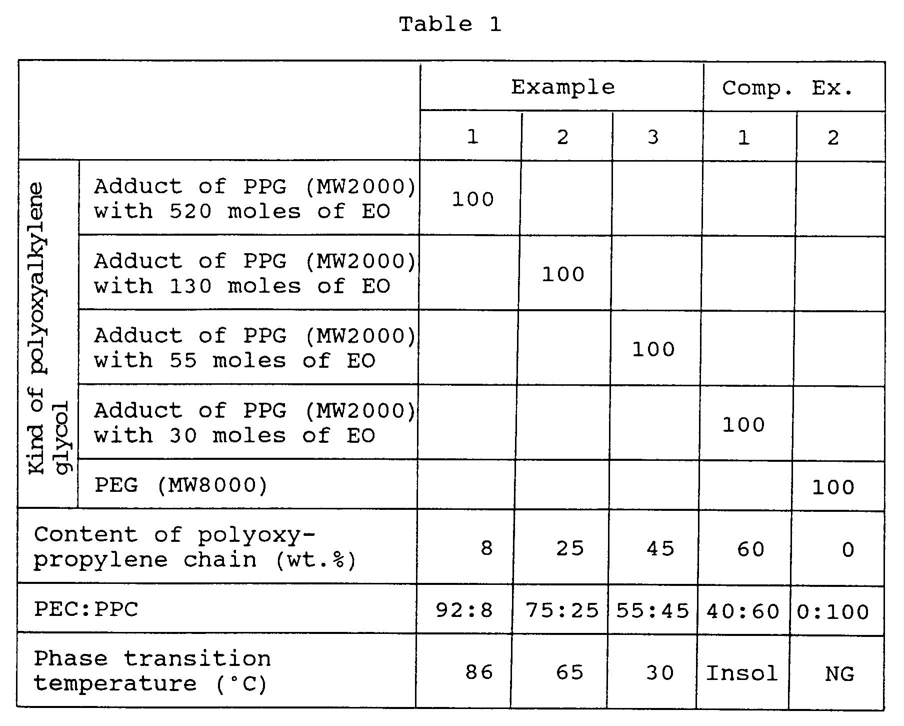

In the rows of "Kind of polyoxyalkylene glycol", PPG and PEG mean polyoxypropylene glycol and polyoxyethylene glycol, respectively. NG in the row of "Phase transition temperature" indicates that the aqueous solution did not gel even when heated to 95°C.

### Examples 4-6 and Comparative Examples 3-4:

Various kinds of polyoxypropylene glycols having an average molecular weight of 500-30,000 shown in Table 2 and polyoxyethylene glycol having an average molecular weight of 9,000 were used. The respective polyoxypropylene glycols and the polyoxyethylene glycol were mixed with each other in a weight ratio of 10:90, and the resultant mixtures were used as polyoxyalkylene glycols (the content of polyoxypropylene chain(s): 10 wt.%). Respective 300-ml four-necked flasks were charged with 100 g of their corresponding polyoxyalkylene glycols, tolylene diisocyanate in an amount of 1 molar equivalent to hydroxyl groups in the polyoxyalkylene glycol, 50 mg of an antioxidant (IRGANOX 245) and 50 mg of dibutyltin dilaurate to conduct reaction 60°C for 30 hours, thereby obtaining respective polyether polyurethanes. Water was added to the resultant polyether polyurethanes to prepare 5% aqueous solutions. Each of the aqueous solutions was heated in the same manner as in Example 1-3 and Comparative Examples 1-2 to measure a phase transition temperature. Incidentally, the polyether polyurethane obtained in Comparative Example 3 was insoluble in water of 0°C or higher, and so no aqueous solution was obtained.

The compositions of the polyoxyalkylene glycols, the phase transition temperatures of the resulting polyether polyurethanes and the weight ratios (PEC:PPC) of PEC to PPC in the polyether polyurethanes are shown in Table 2.

From all the polyether polyurethanes obtained in Examples 4-6, aqueous solutions, which were transparent and fluid at room temperature, were obtained. These solutions exhibited a clear gel state when heated beyond their phase transition temperatures, respectively. When the gelled solutions were cooled to room temperature, all of them returned to the state of fluid solution before the heating. Besides, the aqueous solutions were recognized to show a tendency for the phase transition temperature to lower as the average molecular weight of the polyoxypropylene glycol increased. To the contrary, the aqueous solution of the polyether polyurethane of Comparative Example 4 obtained from the polyoxypropylene glycol having an average molecular weight of 500 did not gel even when heated to 95°C or higher.

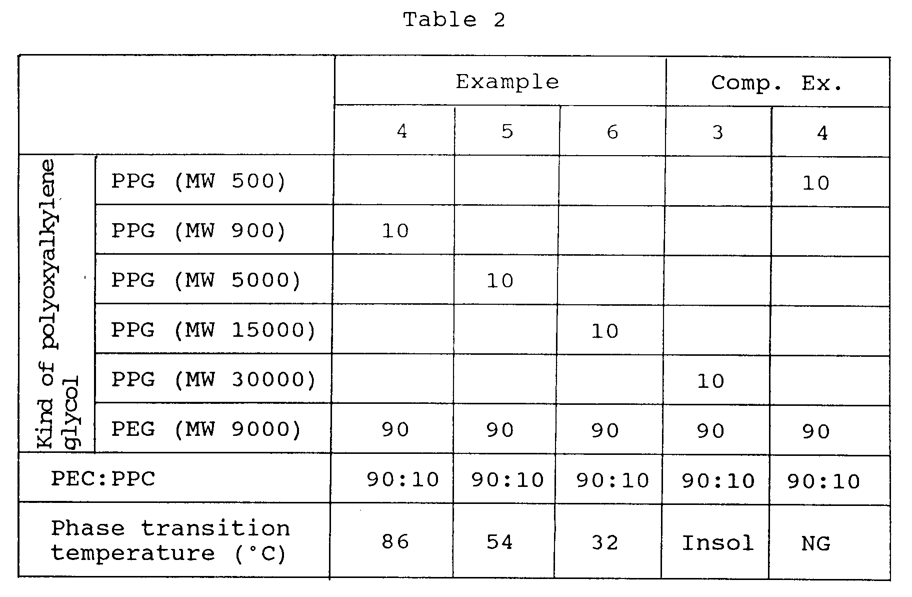

In the rows of "Kind of polyoxyalkylene glycol", PPG and PEG mean polyoxypropylene glycol and polyoxyethylene glycol, respectively. NG in the row of "Phase transition temperature" indicates that the aqueous solution did not gel even when heated to 95°C.

### Examples 7-9 and Comparative Examples 5-6:

Polyoxyethylene-polyoxypropylene glycol [containing 20 wt.% of polyoxypropylene chain(s)] obtained by adding propylene oxide (hereinafter abbreviated as "PO") to polyoxyethylene glycol having an average molecular weight of 16,000 was used as a polyoxyalkylene glycol. Respective 300-ml four-necked flasks were charged with 100 g of the polyoxyalkylene glycol, hexamethylene diisocyanate in their corresponding amounts of 0.3-1.7 molar equivalents to hydroxyl groups in the polyoxyalkylene glycol, 50 mg of an antioxidant (IRGANOX 245) and 50 mg of dibutyltin dilaurate to conduct reaction 60°C for 30 hours, thereby obtaining respective polyether polyurethanes. Water was added to the resultant polyether polyurethanes to prepare 5% aqueous solutions. Each of the aqueous solutions was heated in the same manner as in Example 1-3 and Comparative Examples 1-2 to measure a phase transition temperature. Incidentally, the polyether polyurethane obtained in Comparative Example 6 was not dissolved in water at all.

The numbers of moles of hexamethylene diisocyanate used in the reactions per mole of the polyoxyethylenepolyoxypropylene glycol, the phase transition temperatures of the resulting polyether polyurethanes and the weight ratios (PEC:PPC) of PEC to PPC in the polyether polyurethanes are shown in Table 3.

From all the polyether polyurethanes obtained in Examples 7-9, aqueous solutions, which were transparent and fluid at room temperature, were obtained. These solutions exhibited a clear gel state when heated beyond their phase transition temperatures, respectively. When the gelled solutions were cooled to room temperature, all of them returned to the state of fluid solution before the heating. To the contrary, the aqueous solution of the polyether polyurethane of Comparative Example 5 obtained by reacting 1 mole of the polyoxyalkylene glycol with 0.3 mole of hexamethylene diisocyanate did not gel even when heated to 95°C or higher.

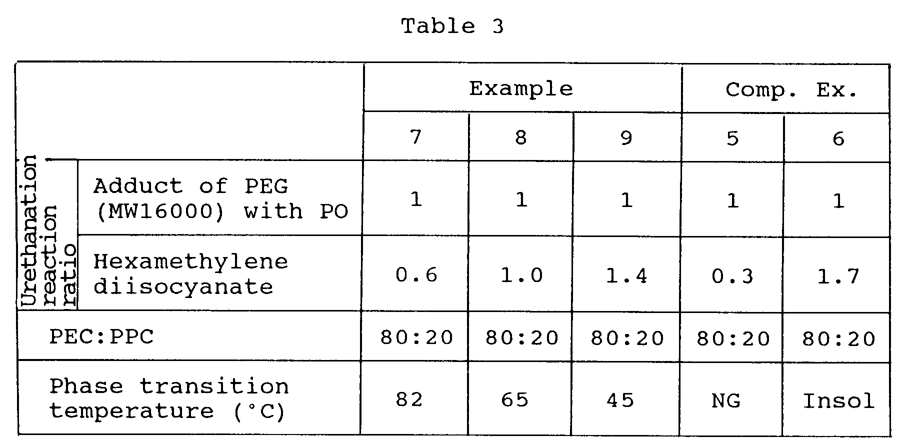

PEG (MW16000) means polyoxyethylene glycol having an average molecular weight of 16,000. NG in the row of "Phase transition temperature" indicates that the aqueous solution did not gel even when heated to 95°C.

### Examples 10-12 and Comparative Example 7:

The polyether polyurethanes obtained in Examples 2, 6 and 7 and Comparative Example 4 were separately used to prepare 5% aqueous solutions, thereby determining the relationship between temperature and viscosity in each of the aqueous solutions. The results are shown in Table 4. All the aqueous solutions in Examples 10, 11 and 12 exhibited good viscosity increase and gelation as their temperature rose. This suggests that these polyether polyurethanes are suitable for thickeners for water-based paints and the like, printing pastes, and the like. On the other hand, the aqueous solution in Comparative Example 7 decreased in viscosity as its temperature rose and was unsuitable for a thickener.

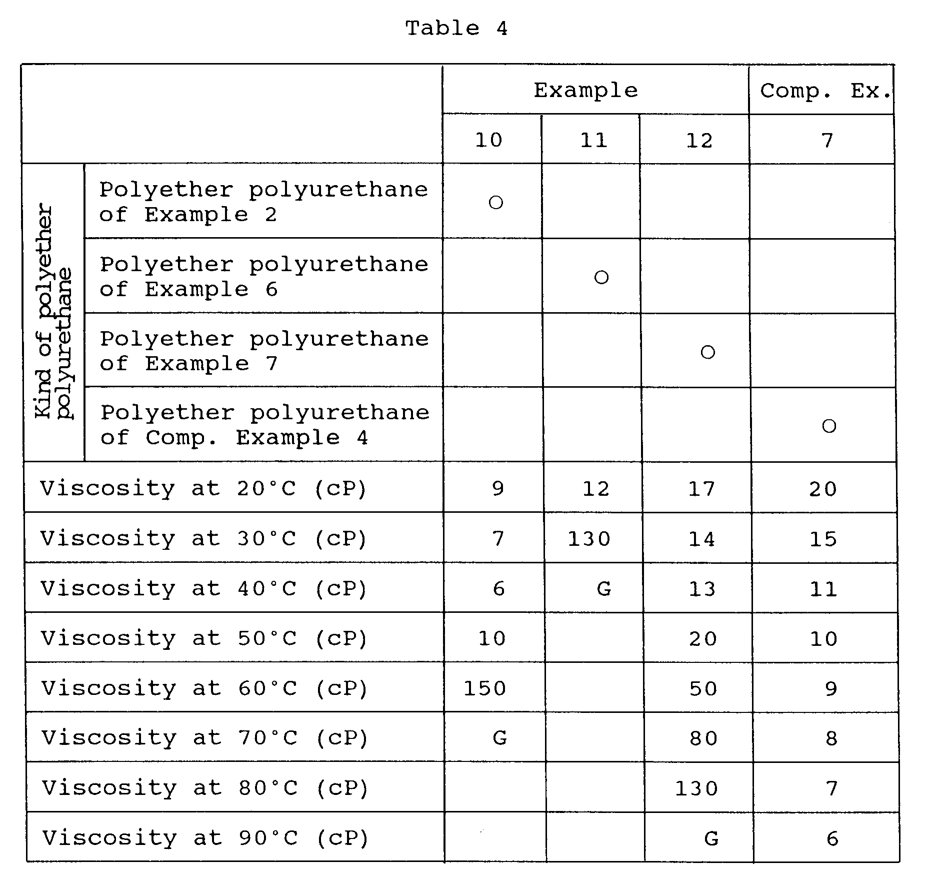

In the table, G indicates that the aqueous solution gelled (became a state that its viscosity was unmeasurable).

### Example 13:

A 5% aqueous solution of the polyether polyurethane obtained in Example 3 was heated from 20°C to 60°C at a heating rate of 0.5°C/min to determine the relationship between temperature and transmittance (at 550 nm) by means of a spectrophotometer equipped with a temperature controller (UV-2200, manufactured by Shimadzu Corporation). The results are shown in Table 5. As shown in Table 5, the aqueous solution of this polyether polyurethane started to gradually reduce its transmittance from the point where the temperature rose beyond 40°C, and the transmittance was 0% at 60°C. When the heated aqueous solution was cooled, the aqueous solution returned to the state of fluid solution and the transmittance before the heating. This suggests that the polyether polyurethane obtained in Example 3 is useful as a light-screening material.

**Table 5**

| Temperature (°C) | Transmittance (%) |
|---|---|
| 20 | 100 |
| 25 | 100 |
| 30 | 100 |
| 35 | 100 |
| 40 | 100 |
| 45 | 88 |
| 50 | 76 |
| 55 | 43 |
| 60 | 0 |

## Claims

1. A thermosensitive polyether polyurethane obtained from a polyoxyalkylene glycol and a diisocyanate compound, characterized in that the polyurethane contains polyoxyethylene chain(s), and polyoxypropylene chain(s) having an average molecular weight of 700-20,000 in a weight ratio of 95:5 to 50:50.

2. The thermosensitive polyether polyurethane according to Claim 1, wherein the diisocyanate compound is an aliphatic diisocyanate and/or a alicyclic diisocyanate.

3. A method of producing a thermosensitive polyether polyurethane, characterized in that polyoxyethylene-polyoxypropylene glycol containing polyoxyethylene chain(s), and polyoxypropylene chain(s) having an average molecular weight of 700-20,000 in a weight ratio of 95:5 to 50:50 is reacted with a diisocyanate compound in such an amount that isocyanate groups amount to 0.5-1.5 molar equivalents to hydroxyl groups in the polyoxyethylene-polyoxypropylene glycol.

4. A method of producing a thermosensitive polyether polyurethane, characterized in that a mixture of polyoxyethylene glycol, and polyoxypropylene glycol having an average molecular weight of 700-20,000 mixed in such amounts that a weight ratio of polyoxyethylene chain(s) to polyoxypropylene chain(s) in the mixture is 95:5 to 50:50 is reacted with a diisocyanate compound in such an amount that isocyanate groups amount to 0.5-1.5 molar equivalents to hydroxyl groups in the mixture.

5. A method of producing a thermosensitive polyether polyurethane, characterized in that a mixture of polyoxyethylene-polyoxypropylene glycol containing polyoxypropylene chain(s) having an average molecular weight of 700-20,000, and polyoxyethylene glycol and/or polyoxypropylene glycol having an average molecular weight of 700-20,000 mixed in such amounts that a weight ratio of polyoxyethylene chain(s) to polyoxypropylene chain(s) in the mixture is 95:5 to 50:50 is reacted with a diisocyanate compound in such an amount that isocyanate groups amount to 0.5-1.5 molar equivalents to hydroxyl groups in the mixture.

6. A thermosensitive composition comprising an aqueous solution of the thermosensitive polyether polyurethane according to Claim 1 or 2 as a principal component.
